Europäisches Patentamt

European Patent Office

Office européen des brevets

(19)

(11) Veröffentlichungsnummer: **0 263 328**

**A1**

(12) **EUROPÄISCHE PATENTANMELDUNG**

(21) Anmeldenummer: 87113428.4

(22) Anmeldetag: 15.09.87

(51) Int. Cl.4: **C12N 15/00** , C12N 5/00 , A61K 37/02

Der Mikroorganismus ist bei der European Collection of Animal Cell Cultures (ECACC) unter der Nummer 86091105 hinterlegt worden.

(30) Priorität: 20.09.86 DE 3632038

(43) Veröffentlichungstag der Anmeldung:
13.04.88 Patentblatt 88/15

(84) Benannte Vertragsstaaten:
AT BE CH DE ES FR GB GR IT LI LU NL SE

(71) Anmelder: **BEHRINGWERKE Aktiengesellschaft Postfach 1140 D-3550 Marburg 1(DE)**

(72) Erfinder: **Pawlita, Michael, Dr. Finkenweg 1 D-6925 Eschelbronn(DE)**
Erfinder: **Zur Hausen, Harald, Prof. Dr. Dr. Berliner Strasse 38a D-6900 Heildelberg(DE)**

(74) Vertreter: **Meyer-Dulheuer, Karl-Hermann, Dr. et al HOECHST Aktiengesellschaft Zentrale Patentabteilung Postfach 80 03 20 D-6230 Frankfurt/Main 80(DE)**

(54) **Gewebespezifische virale Vektoren auf Basis des LPV und ihre Verwendung.**

(57) Vektoren, die den Ursprung der DNA-Replikation und im wesentlichen die späte Genregion des lymphotropen Papova-Virus (LPV) enthalten, eignen sich zur Transfektion von LPV-permissiven Zellen, die die frühe Genregion von LPV stabil exprimieren. In die Vektoren eingebaute Fremd-DNA wird dann in diesen Zellen repliziert und in LPV-Partikel verpackt. Unter Ausnutzung der Wirtsspezifität des LPV für B-Lymphomzellen kann die Fremd-DNA in Zellen durch Infektion mit diesen replikationsdefekten Partikeln eingeführt und exprimiert werden. Wenn die Fremd-DNA für ein Protein codiert, das für die infizierten Zellen toxisch ist, kann dieses Vektorsystem zur Therapie von B-Lymphomen verwendet werden.

EP 0 263 328 A1

## Gewebespezifische virale Vektoren auf Basis des LPV und ihre Verwendung

Das lymphotorope Papovavirus (LPV) wird nur in Zellinien propagiert, die sich von B-Lymphomen ableiten, beispielsweise die BJA-B-Zellinie, die von einem menschlichen malignen B-Lymphom stammt (Menezes et al., Biomedicine 22 (1975) 276-284) oder in sehr geringem Maße in B-lymphoblastoiden Zellinien (B-Lymphozyten, die durch das Epstein-Barr-Virus immortalisiert sind, Pawlita et al., Virology 143 (1985) 196-211, und darin genannte Literatur).

Diese Gewebespezifität ist eine Eigenschaft des Viruspartikels, da isolierte virale DNA nach Transfektion auch in nicht-permissiven Zellen exprimiert wird. Hieraus ergibt sich also die Möglichkeit, dieses hochspezifische Virus oder einen Vektor auf Basis dieses Virus zur Einführung von für Toxine oder andere Wirkstoffe codierenden DNA-Sequenzen in maligne B-Zellen einzubringen (Mechanisms of B Cell Neoplasia 1985, Editiones Roche, Basel, S. 274-278).

Die Erfindung ist in den Patentansprüchen definiert. Weitere Ausgestaltungen werden im folgenden näher beschrieben.

LPV ist bei der American Type Culture Collection unter der Nummer ATCC VR 961 hinterlegt.

Die gesamte DNA-Sequenz eines LPV-Klones (LPV K38) ist in Pawlita et al., a.a.O., wiedergegeben. Das dort genannte Plasmid pLPV K38 enthält das gesamte Genom, geöffnet an der einzigen BamHI-Schnittstelle, ligiert in die BamHI-Stelle von pBR322. Dieses Plasmid eignet sich als Ausgangsmaterial für die Herstellung der erfindungsgemäßen Vektoren und Wirtszellen.

Es ist natürlich auch möglich, auf Basis der bekannten LPV-DNA-Sequenz in an sich bekannter Weise die erfindungsgemäß eingesetzten LPV-DNA-Segmente zu synthetisieren, gegebenenfalls unter Verwendung homologer Segmente anderer Viren (Pawlita et al., a.a.O.).

Die Figuren zeigen in Form eines Fließschemas die Herstellung von Vektoren bzw. von LPV-DNA-Segmenten, mit denen eukaryotische Zellen transfiziert werden. Die Figuren sind nicht maßstabsgerecht gezeichnet, insbesondere im Bereich der Polylinker ist der Maßstab gedehnt. Die LPV-DNA ist durch dicke Linien hervorgehoben.

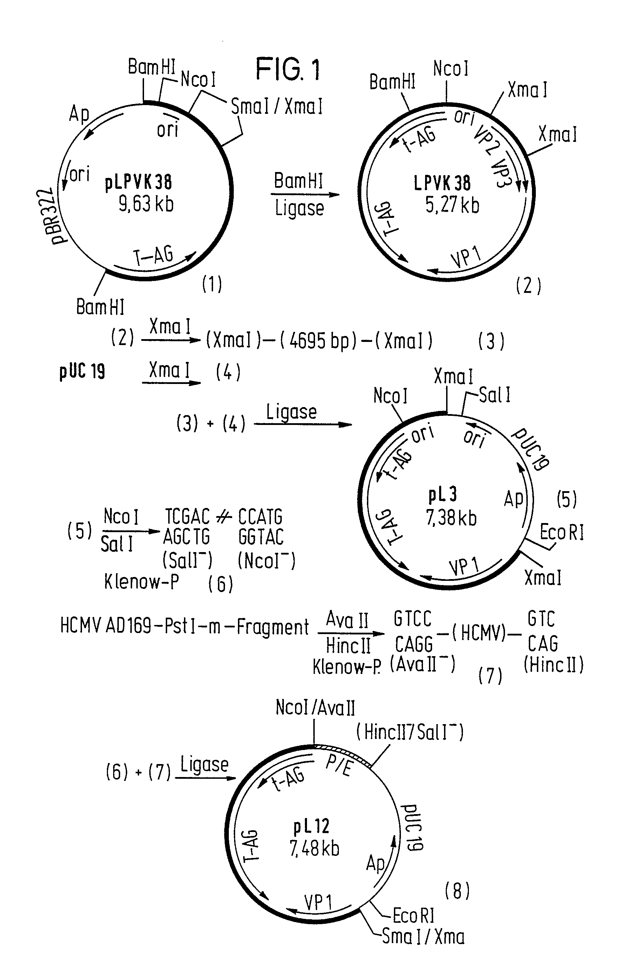

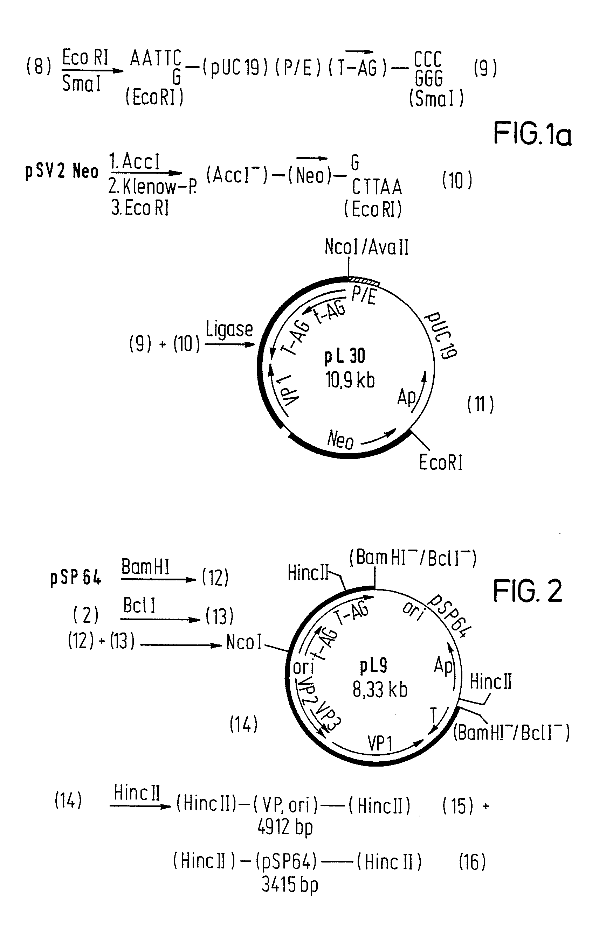

Figur 1 und ihre Fortsetzung, Figur 1a, zeigen die Synthese des Plasmids pL30, das die frühe Genregion von LPV unter Kontrolle eines fremden Promotors sowie als Marker eine Neomycinresistenz enthält. Dieses Plasmid eignet sich zur Transfektion einer permissiven Zellinie, wobei in hoher Ausbeute Zellen erhalten werden, die LPV-T-Antigen exprimieren.

Die Figur 2 zeigt die Synthese des Plasmids pL9, das nach Spaltung mit HincII ein etwa 4,9 kb-Fragment ergibt, das auf Grund seiner Größe in virale Partikel verpackt werden kann. Bringt man dieses DNA-Fragment in die erzeugten LPV-T+-Zellen, so werden infektiöse, aber replikationsdefekte Viruspartikel gebildet.

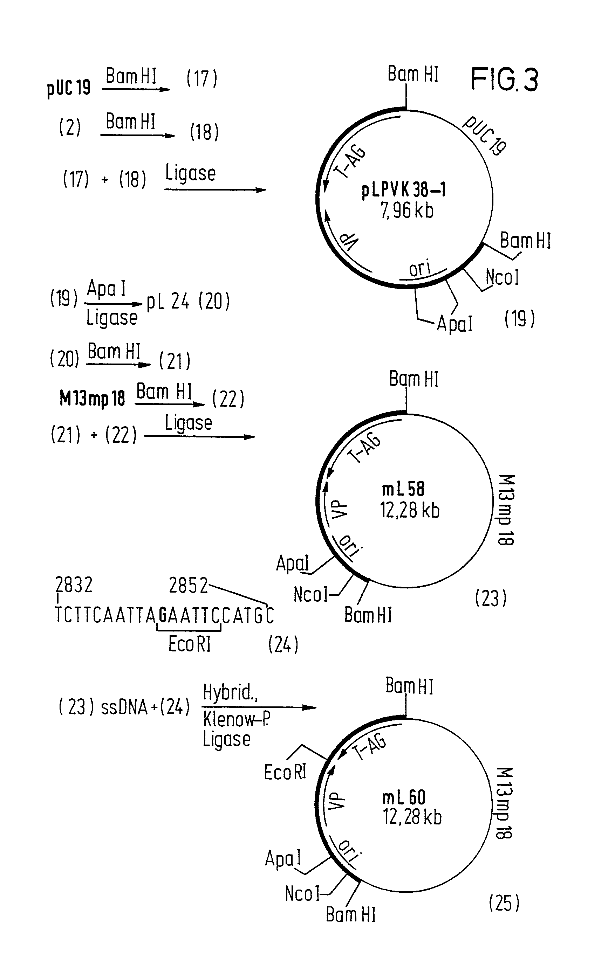

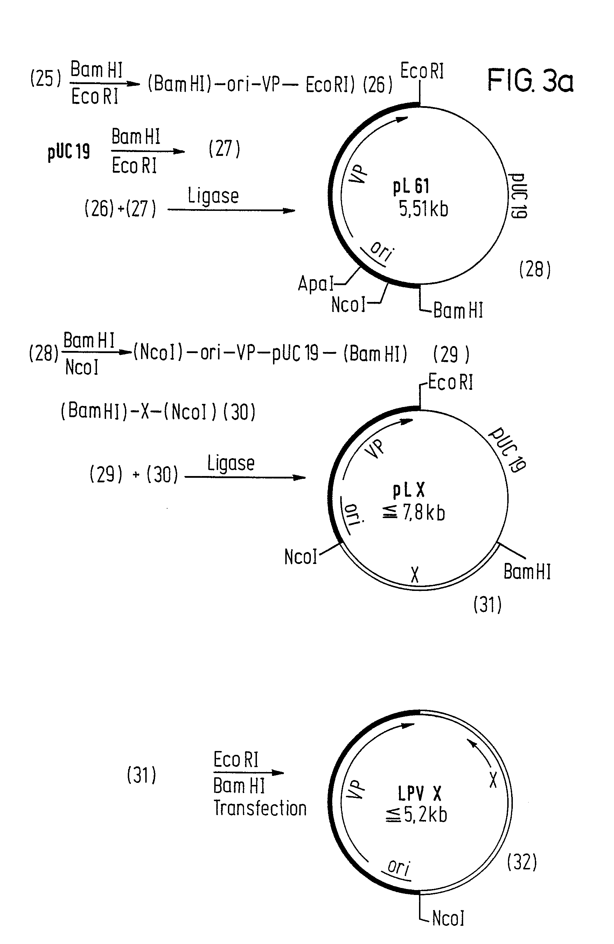

Die Figur 3 und ihre Fortsetzung, Figur 3a, zeigen die Synthese von Vektoren, die Fremd-DNA exprimieren können. Hierzu wird zunächst der Vektor pL61 synthetisiert, der den DNA-Replikationsursprung und die späte Genregion von LPV enthält. Durch Ersatz des pUC19-Anteils durch das gewünschte Gen erhält man den Vektor LPV X, der in den erfindungsgemäßen Zellen, die die frühe Genregion von LPV stabil exprimieren, zur Replikation und Verpackung des Fremdgens X in defekte, infektiöse Viruspartikel führt.

Zur Konstruktion des LPV-Vektors wurde das von Gluzman, Cell 23 (1981) 175-182 (SV40-COS-System) beschriebene Prinzip des "early replacement vector" angewandt. Im Genom von LPV codiert die "frühe" Genregion für ein Protein (T-Antigen), das zur Replikation der viralen DNA und zur Expression der "späten", für die Viruscapsidproteine VP1, VP2 und VP3 codierenden Genregion notwendig ist. Die erfindungsgemäß als Vektoren verwendeten replikationsdefekten LPV-Partikel tragen in ihrem Genom statt der frühen Genregion fremde DNA-Sequenzen. Bei der Synthese dieser defekten Partikel ist die Anwesenheit des LPV-T-Antigens in den synthetisierenden Zellen notwendig, nämlich die erfindungsgemäßen LPV-permissiven Zellen, die die frühe Genregion von LPV stabil exprimieren.

Das im folgenden beschriebene vorteilhafte Verfahren zur Herstellung von stabil LPV-T-Antigen exprimierenden Zellinien sowie zur Herstellung infektiöser, replikationsdefekter LPV-Partikel, die effizient und spezifisch Fremd-DNA in LPV-suszeptible Zellen transportieren und dort zur Expression bringen können, kann selbstverständlich von einem Fachmann auch in abgewandelter Weise durchgeführt werden, beispielsweise mit Hilfe anderer Genomfragmente, anderen Zellinien und anderen Fremd-DNA-Fragmenten.

Als LPV-suszeptible humane B-Lymphomzellinie wurde die etablierte Zellinie BJA-B (Menezes et al., a.a.O.) eingesetzt, da diese sich effektiv transfizieren läßt. Diese Zellinie ist erhältlich bei der European Collection of Animal Cell Cultures (ECACC), Porton Down, Salisbury (GB), hinterlegt gemäß den Bedingungen des Budapester Vertrags unter der Nummer 86081105.

Da in dem Ausgangsplasmid pLPV K38 die BamHI-Restriktionsschnittstelle in der "frühen" Genregion lokalisiert ist, muß zunächst virale DNA gewonnen werden, deren frühe, für das LPV-T-Antigen codierende Region nicht unterbrochen ist. Hierzu wird die Plasmid-DNA aus pLPV K38 mit BamHI herausgespalten und zur Zirkularisierung der LPV-DNA ligiert. Mit dieser ligierten DNA werden BJA-B-Zellen transfiziert und das in dieser Kultur synthetisierte LPV K38 als Impfvirus zur Herstellung größerer Mengen von LPV-DNA verwendet.

Aus der LPV K38-DNA wird das 4695 bp große XmaI-Restriktionsfragment in die XmaI-Restriktionsschnittstelle des bakteriellen Vektors pUC19 einkloniert. Das erhaltene Plasmid pL3 enthält die gesamte frühe Genregion und den 3'-terminalen Teil der späten Genregion, die aber wegen des Verlustes der späten Promotorregion und der 5'-terminalen Sequenzen nicht exprimiert werden kann. Zur Deletion des Ursprungs der LPV-DNA-Replikation wird die verbliebene, nicht codierende LPV-Kontrollregion (Nucleotid 1 bis 528) durch ein Promotor-Enhancerelement aus der "immediate early"-Region des humanen Cytomegalovirus (HCMV) ersetzt (Europäische Patentanmeldung mit der Veröffentlichungsnummer 0 173 177). Hierzu wird das Plasmid pL3 mit den Restriktionsenzymen NcoI und SalI verdaut und die 5'-überstehenden Enden mit Hilfe des Klenow-Fragmentes der DNA-Polymerase I aus E. coli aufgefüllt. Aus der HCMV-DNA (PstI-m-Fragment) wird das 650 bp große AvaII-HincII-Restriktionsfragment (Nucleotid -598 bis +52) isoliert und nach Auffüllen des 5'-überstehenden Endes in das vorbereitete Fragment des Plasmids pL3 einkloniert, wobei das Plasmid pL12 erhalten wird. Die richtige Orientierung des HCMV-Promotor-EnhancerElementes in bezug auf die frühe LPV-Genregion ergibt sich einerseits aus der Rekonstitution der NcoI-AvaII-Schnittstelle und andererseits durch Verdauung mit BglI auf Grund der beiden Schnittstellen für dieses Restriktionsenzym in der pUC19-DNA und den BglI-Schnittstellen in dem HCMV-Fragment.

Das Plasmid pSV2Neo (Southern und Berg, J. Mol. Appl. Gen. 1 (1982), 327-341) enthält das prokaryote Neo-Gen zusammen mit drei eukaroyten Regulationselementen, nämlich den frühen Enhancer-Promoter, eine RNA-Spleiß-Sequenz und ein Polyadenylierungssignal des SV40-Genoms. Eukaryote Zellen, in denen dieses Gen exprimiert wird, sind resistent gegen das dem Neomycin verwandte, toxische Aminoglycosid-Antibiotikum G 418. Durch Spaltung mit AccI, Auffüllen der überstehenden 5'-Enden und anschließende Spaltung mit EcoRI isoliert man aus PSV2Neo das 2867 bp große Fragment, das in das mit EcoRI und SmaI verdaute Plasmid pL12 einkloniert wird. Hierbei wird das Plasmid pL30 erhalten.

Das Plasmid pL30 wird mit EcoRI linearisiert und mit dieser DNA die Zellinie BJA-B transfiziert. Durch Selektion auf Resistenz gegen G 418 werden Neo$^r$-Zellklone identifiziert und durch Subkultivierung isoliert. Durch indirekte Immunfluoreszenz mit einem anti-LPV-T-Serum können LPV-T-Antigen exprimierende Zellinien identifiziert werden. Die so erhaltenen BJA-B (Neo$^r$ LPV-T$^+$)-Linien werden kultiviert und einer dieser Zellklone (31B3-K6) für die weiteren Experimente verwendet.

Zur Herstellung defekter Viruspartikel wird das LPV K38-Genom über die BclI-Restriktionsschnittstelle (Nucleotid 3328) in die BamHI-Restriktionsschnittstelle des Plasmids pSP64 kloniert, wobei man das Plasmid pL9 erhält. Mittels dieser pSP64-Insertion ist über die Inaktivierung der frühen LPV-Region sichergestellt, daß durch pL9 kein funktionelles T-Antigen synthetisiert wird. Eine Transfektion der 31B3-K6-Zellen mit dem 8.27 kb großen Plasmid pL9 in geschlossener, zirkulärer Form ergibt 48 Stunden nach Transfektion 0,1 % LPV-VP$^-$-Zellen, deren Anteil danach wieder abfällt. In zu Kontrollzwecken transfizierten BJA-B-Zellen ist keine LPV-VP-Synthese nachweisbar. Hierdurch ist bewiesen, daß das in den 31B3-K6-Zellen vorhandene LPV-T-Antigen funktionell ist.

Da das Plasmid pL9 wegen seiner Größe nicht in LPV-Viruspartikel verpackbar ist, wird es mit der Restriktionsendonuclease HincII in ein 3415 bp großes Fragment gespalten, das neben der pSP64-Sequenz noch 370 bp der frühen LPV-Genregion enthält (Nucleotid 3328 bis 3698) sowie ein 4912 bp großes Fragment, das 4900 bp der LPV-DNA-Sequenz (Nucleotid 3698 bis 3327) neben 12 bp der pSP64-DNA-Sequenz umfaßt. Das 4912 bp große Fragment enthält neben der nicht codierenden LPV-Kontrollregion mit dem Ursprung der LPV-DNA-Replikation die komplette späte LPV-Region und Teile der frühen LPV-Region, die aber kein LPV-T-Antigen mehr codieren können.

pL9-Plasmid-DNA wird mit HincII verdaut und damit werden 31B3-K6-Zellen transfiziert. 48 Stunden nach der Transfektion erhält man ebenfalls 0,1 % LPV-VP$^+$-Zellen. Im Gegensatz zu den mit unverdautem pL9-Plasmid erhaltenen Zellen stieg der Anteil der LPV-VP$^+$-Zellen innerhalb von sechs weiteren Inkubationstagen auf über 50 % an, was auf die Produktion infektiöser LPV-Viruspartikel hinweist. Werden BJA-B-Zellen und 31B3-K6-Zellen mit steril filtrierten Kulturüberständen behandelt, so erhält man 48 Stunden nach Beimpfen in den 31B3-K6-Zellen 10 % LPV-VP$^+$-Zellen, während die beimpften BJA-B-Zellen auch nach acht Tagen negativ bleiben. Hierdurch wird die Existenz infektiöser, aber replikationsdefekter LPV-Partikel bewiesen.

Das LPV-Capsid kann ein bis zu 5,3 kb großes Genom beherbergen. Die nicht codierende Kontrollregion und die späte Genregion, die beide zur Herstellung defekter Partikel im Genom benötigt werden, umfassen zusammen 2605 bp. Zur Einklonierung von Fremd-DNA verbleiben somit noch maximal etwa 2,7 kb, falls alle Sequenzen der frühen Genregion deletiert werden.

Nach Einführen einer EcoRI-Restriktionsschnittstelle an Position 2843 durch gezielte in vitro-Mutagenese (Grundström et al., Nucleic Acids Res. 13 (1985) 3305-3316) kann die gesamte frühe LPV-Genregion durch Doppelverdauung mit den Restriktionsendonucleasen NcoI (Position 5268) und EcoRI herausgeschnitten werden.

Hierzu wird zunächst der Vektor pUC19 mit BamHI geöffnet und mit dem ebenfalls mit BamHI geöffneten LPV K38-Genom ligiert, wobei das Plasmid pLPV K38-1 erhalten wird. Aus diesem wird durch Schneiden mit ApaI das 240 bp umfassende Fragment eliminiert und das Restplasmid religiert, wobei das Plasmid pL24 erhalten wird. Aus pL24 wird durch Schneiden mit BamHI die virale DNA isoliert und über die BamHI-Restriktionsschnittstelle in den Vektor M13mp18 (New England Biolabs) kloniert, wobei der Vektor mL58 erhalten wird. Das synthetische 21 bp große Oligonucleotid
TCTTCAATTAGAATTCCATGC,
das die LPV-Sequenz von Nucleotid 2832 bis 2852 mit einem Guanosin-statt einem Cytosin-Rest in Position 11 (LPV 2842) enthält, wird an die Einzelstrang-Phagen-DNA hybridisiert und als Primer für die Synthese des komplementären DNA-Stranges mit dem Klenow-Fragment benutzt. Mutagenisierte M13-Klone mit EcoRI-Restriktionsschnittstellen (mL60) werden durch Restriktionsanalyse der replikativen Form identifiziert. Aus diesen wird durch Schneiden mit BamHI und EcoRI das 2826 bp große Fragment isoliert und über die entsprechenden Schnittstellen in pUC19 einkloniert, wobei das Plasmid pL61 erhalten wird. Dieses enthält neben der kompletten späten LPV-Genregion und dem Ursprung der LPV-DNA-Replikation auch das Promotor-Enhancer-Element und die Polyadenylierungs-Signalsequenzen der frühen LPV-Genregion, so daß nur die codierenden Sequenzen eines fremden Genes X in das durch Doppelverdauung mit den Restriktionsendonucleasen NcoI und BamHI geöffnete Plasmid einkloniert werden müssen, um ein funktionelles, exprimierbares, eukaryotes Gen zu erhalten (Plasmid pLX).

Als einzuführende Testgene eignen sich hierbei insbesondere solche Sequenzen, die für ein leicht meßbares und quantifizierbares Enzym codieren wie Teile der β-Galaktosidase oder Chloramphenicol-Acetyl-Transferase. Nach Doppelverdauung des Plasmids pLX mit den Restriktionsendonucleasen EcoRI und BamHI und anschließender Transfektion in 31B3-K6-Zellen werden defekte Viruspartikel LPV X gebildet, mit denen LPV-susceptible Zellinien wie BJA-B oder Namalwa infiziert werden. Die Expression der Fremd-DNA kann dann in einer enzymatischen Reaktion in Proteinextrakten der infizierten Zellen nachgewiesen werden. Als weitere Testgene eignen sich DNA-Sequenzen, die für gut charakterisierte Antigene codieren wie Hepatitis B-Oberflächen-Antigen (HBs-Ag) und deren Expression durch Antikörperreaktionen leicht nachweisbar ist.

Die erfindungsgemäße Nutzung der hohen Gewebespezifität kann dadurch erfolgen, daß das eingeführte Fremdgen für ein Polypeptid codiert, das für die Wirtszelle toxisch ist oder antitumoral wirkt. So kann beispielsweise das A-Peptid des Diphtherietoxins zum spezifischen Abtöten von LPV-permissiven Lymphomzellen verwendet werden. Eine weitere Möglichkeit stellt die Expression von Antisense-RNA gegen aktivierte Onkogene der Antitumorale Effekte können auch durch die Expression von membrangebundenen Antigenen, gegen die gute Immunität besteht, hervorgerufen werden.

Denkbar ist auch ein Einsatz des erfindungsgemäßen Vektorsystems zur Immunisierung von Nicht-Tumor-Patienten, da vermutlich auch bestimmte normale B-Zell-Differenzierungsstufen susceptibel für LPV-Infektionen sind. Die Expression neuer, durch die Fremd-DNA codierter Antigene als Membranproteine, beispielsweise HBsAg, kann dazu benutzt werden, Antigen-spezifische, insbesondere zellvermittelte, Immunität zu induzieren. Eine weitere Möglichkeit zur Anwendung der Erfindung liegt in der Elimination gesunden Gewebes, beispielsweise im Sinne einer Immunsuppression.

Die Erfindung wurde vorstehend anhand des LPV und seiner ausgeprägten Gewebespezifität erläutert. Es ist jedoch bekannt, daß auch andere humane Viren eine Gewebespezifität aufweisen, so beispielsweise die beiden humanen Retroviren HTLV I und LAV/HTLV III, die mit hoher Präferenz Zellen der T-Lymphozytenreihe befallen. Humane Papillomviren (HPV) wurden bisher ausschließlich aus epithelialen Zellen der Epidermis oder der Mucosa isoliert. Das Epstein-Barr-Virus (EBV) ein humanes Herpes-Virus, konnte bisher nur in B-Lymphozyten und Epithelien des Nasen-Rachen-Raumes nachgewiesen werden. Die Erfindung eröffnet somit auch einen Zugang zum Aufbau weiterer gewebespezifischer Vektorsysteme unter Verwendung anderer gewebespezifischer Viruspartikel.

Die in den folgenden Beispielen beschriebenen Verfahren werden durch die Figuren erläutert, wobei die Bezifferung der Figuren und der Beispiele übereinstimmt.

Beispiel 1

Das Plasmid pLPV K38 (1) wird mit BamHI gespalten und die LPV-DNA zur Zirkularisierung zu LPV K38 (2) ligiert. Nach Reinigung wird die ligierte DNA nach der Methode von McCutchan und Pagano, J. Natl. Cancer Inst. 41 (1968), 351-357, in 600 $\mu$l Dextran-Transfektionspuffer (RPMI 1640-Medium mit 500 $\mu$l/ml DEAE-Dextran vom Molgewicht 500000) zur Transfektion mit 2 x $10^6$ gewaschenen BJA-B-Zellen gemischt und 30 Minuten bei Raumtemperatur inkubiert. Nach einmaligem Waschen werden die transfizierten Zellen in 10 ml Wachstums-Medium (RPMI 1640, 10% fötales Kälberserum) bei 37°C und 5 Vol.-% $CO_2$ inkubiert. Das in dieser Kultur synthetisierte LPV K38 wird als Impfvirus zur Herstellung größerer Mengen von LPV-DNA nach der Methode von Hirt, J. Mol. Biol. 26 (1967), 365-369, verwendet.

Für die folgenden Klonierungen in E. coli (Stamm 490 A) und Plasmid-DNA-Präparierungen werden Standardmethoden angewandt (Maniatis et al., Molecular Cloning, Cold Spring Harbor Laboratory, 1982).

LPV K38 (2) wird mit XmaI verdaut und das 4695 bp große Fragment (Nucleotid 1104 bis 529) (3) isoliert. Der Vektor pUC19 wird mit XmaI geöffnet und das linearisierte Plasmid (4) mit dem Fragment (3) ligiert, wobei das Plasmid pL3 (5) erhalten wird.

Das Plasmid (5) wird mit NcoI and SalI verdaut und die überstehenden Enden mit Klenow-Polymerase aufgefüllt, wobei das stumpfendige Fragment (6) erhalten wird.

Das HCMV AD169-PstI-m-Fragment (EP-A1-0 173 177) wird mit AvaII und HincII verdaut und das 650 bp große Fragment (Nucleotid -598 bis +52) isoliert. Durch Auffüllen mit Klenow-Polymerase wird das stumpfendige Fragment (7) erhalten.

Die beiden Fragmente (6) und (7) werden unter "blunt end"-Bedingungen ligiert, wobei das Plasmid pL12 (8) erhalten wird. Die richtige Orientierung des HCMV-Promotor-Elementes in bezug auf die frühe LPV-Genregion wird durch Restriktionsanalyse überprüft: Das gewünschte Plasmid enthält eine NcoI-Schnittstelle und ergibt mit BglI die erwarteten Schnitte im pUC19-Anteil und im HCMV-Fragment.

Das Plasmid (8) wird mit EcoRI und SmaI geöffnet und das große Fragment (9) isoliert.

Der Vektor pSV2Neo wird mit AccI verdaut, die überstehenden Enden mit Klenow-Polymerase aufgefüllt und mit EcoRI nachgeschnitten, wobei das Fragment (10) erhalten wird. Durch Ligieren der Fragmente (9) und (10) erhält man das Plasmid pL30 (11).

Das Plasmid pL30 (11) wird mit EcoRI linearisiert und damit in 3 ml Dextran-Transfektionspuffer wie vorstehend beschrieben $10^7$ Zellen der Zellinie BJA-B transfiziert. Nach 48 Stunden Inkubation werden die Zellen auf eine Konzentration von 2 x $10^5$ Zellen pro ml verdünnt und nach Zugabe von G 418 zu einer Endkonzentration von 1,2 mg/ml in 150 $\mu$l Kulturen in 96 Loch-Gewebekulturplatten ausgesät. Nach 10 Tagen werden 50% des Kulturüberstandes durch frisches Medium ersetzt. 25 Tage nach Transfektion können Neo$^r$-Zellklone identifiziert und durch Subkultivierung isoliert werden. Nach weiteren 10 Tagen können LPV-T-Antigen exprimierende Zellinien durch indirekte Immunfluoreszenz mit einem anti-LPV-T-Serum identifiziert werden. Die so erhaltenen BJA-B(Neo$^r$ LPV-T$^+$)-Linien werden durch Ausverdünnen kloniert. BJA-B(Neo$^r$ LPV-T$^+$)-Einzelzell-Klone, die nach 20 Tagen Kultur erhalten werden, werden weiterverarbeitet. Einer dieser BJA-B(Neo$^r$ LPV-T$^+$) Zellklone, die Zellinie 31B3-K6, wird für die weiteren Experimente verwendet.

Beispiel 2:

Das handelsübliche Plasmid pSP64 wird mit BamHI geöffnet, wobei das linearisierte Plasmid (12) erhalten wird.

LPV K38 (2) wird mit BclI geöffnet und das linearisierte Genom (13) mit (12) ligiert. Hierbei wird das Plasmid pL9 (14) erhalten. Dieses wird mit HincII verdaut, wobei zwei Fragmente von 4912 bp (15) und 3415 bp (16) erhalten werden.

Die Zellinie 31B3-K6 (Beispiel 1) wird mit der HincII-verdauten pL9-Plasmid-DNA transfiziert (Transfektionsbedingungen wie im Beispiel 1), wobei 48 Stunden nach Transfektion 0.1 % LPV-VP$^+$-Zellen erhalten wurden. Innerhalb von sechs weiteren Inkubationstagen steigt der Anteil der LPV-VP$^+$-Zellen auf über 50% an. Durch Übertragung steril filtrierter Kulturüberstände auf 31B3-K6-Zellen können 48 Stunden nach Beimpfen 10% LPV-VP$^+$-Zellen nachgewiesen werden. Beimpft man mit dem Kulturüberstand BJA-B-Zellen, so können auch nach acht Tagen keine LPV-VP$^+$-Zellen nachgewiesen werden.

Transfiziert man 31B3-K6-Zellen mit unverdautem Plasmid pL9 (14), so resultieren 48 Stunden nach Transfektion 0,1 % LPV-VP⁺-Zellen, deren Anteil dann aber wieder abfällt.

Tranfiziert man zur Kontrolle unter den gleichen Bedingungen BJA-B-Zellen, so ist keine LPV-VP-Synthese nachweisbar.

Beispiel 3

Für die folgenden Arbeiten wird das LPV-Genom zunächst in den Vektor pUC19 umkloniert. Hierzu wird dieser mit BamHI geöffnet, wobei das linearisierte Plasmid (17) erhalten wird.

LPV K38 (2) wird ebenfalls mit BamHI geöffnet und das linearisierte LPV-Genom (18) mit (17) ligiert, wobei das Plasmid pLPV K38-1 (19) erhalten wird.

Zur Deletion des 240 bp großen ApaI-Fragments, das die Duplikation des Ursprungs der DNA-Replikation in LPV K38 enthält, wird (19) mit ApaI geschnitten und das große Fragment zum Plasmid pL24 (20) ligiert.

Durch Schneiden von (20) mit BamHI wird das verkürzte LPV-Genom (21) erhalten, das in den mit BamHI geschnittenen Phagenvektor M13mp18 (22) ligiert wird, wobei der Vektor mL58 (23) erhalten wird.

An die Einzelstrang-DNA von (23) wird das synthetische Oligonucleotid (24) hybridisiert, das als Primer für die Synthese des komplementären DNA-Stranges mit Klenow-Polymerase dient. Mutagenisierte M13-Klone mit der eingeführten EcoRI-Restriktionsschnittstelle werden durch Restriktionsanalyse der Replikativen Form identifiziert. Sie erhalten die Bezeichnung mL60 (25).

mL60 (25) wird mit BamHI und EcoRI verdaut und das 2826 bp große Fragment (26) isoliert. pUC19 wird ebenfalls mit BamHI und EcoRI verdaut und das große Fragment (27) mit (26) ligiert, wobei das Plasmid pL61 (28) erhalten wird.

Das Plasmid pL61 (28) wird mit BamHI und NcoI geschnitten und das große Fragment (29) isoliert. Dieses eignet sich zum Einbau eines bis zu 2,7 kb großen DNA-Fragmentes (30), wobei ein Plasmid pLX (31) erhalten wird. Durch Schneiden von (31) mit EcoRI und BamHI und Transfektion in 31B3-K6-Zellen werden defekte Viruspartikel LPV X (32) gebildet, die das Fremdgen X enthalten.

**Ansprüche**

1. Wirts-Vektorsysteme, gekennzeichnet durch gewebespezifische virale Vektoren, in denen virale DNA durch Fremd-DNA ersetzt ist und permissive Wirtszellen mit konstitutiver Expression dieser deletierten DNA.

2. Wirts-Vektorsystem nach Anspruch 1, dadurch gekennzeichnet, daß der gewebespezifische virale Vektor den Ursprung der DNA-Replikation und im wesentlichen die späte Genregion des lymphotropen papova-Virus (LPV) umfaßt und LPV-permissive Zellen, die die frühe Genregion von LPV stabil exprimieren, als Wirt fungieren.

3. Verfahren zur Expression von Fremd-DNA, dadurch gekennzeichnet, daß diese Fremd-DNA virale DNA in einem Wirts-Vektorsystem nach Anspruch 1 ersetzt.

4. Verfahren nach Anspruch 3, dadurch gekennzeichnet, daß die Fremd-DNA für ein Protein kodiert, das für die Wirtszelle toxisch ist.

5. Verfahren zur Herstellung eines Vektors für ein Wirts-Vektorsystem nach Anspruch 1, dadurch gekennzeichnet, daß in das Genom von LPV eine singuläre Schnittstelle zwischen früher und später Genregion eingeführt wird und das etwa 3 kb umfassende Segment von der NcoI-Schnittstelle bis zu der eingeführten Schnittstelle verwendet wird.

6. Verfahren zur Herstellung von Wirtszellen für ein Wirts-Vektorsystem nach Anspruch 1, dadurch gekennzeichnet, daß man eine LPV-permissive Zellinie mit einem LPV-DNA-Fragment transfiziert, das das 4,2 kb NcoI-Segment umfaßt.

7. Arzneimittel, gekennzeichnet durch einen Gehalt an einem Vektor nach Anspruch 1, der für ein für die Wirtszelle toxisches Protein kodiert.

Patentansprüche für die folgenden Vertragsstaaten: GR, AT und ES

1. Verfahren, bei dem Wirts-Vektorsysteme zur Anwendung kommen, gekennzeichnet durch gewebespezifische virale Vektoren, in denen virale DNA durch Fremd-DNA ersetzt ist und permissive Wirtszellen mit konstitutiver Expression dieser deletierten DNA.

2. Verfahren nach Anspruch 1, dadurch gekennzeichnet, daß der gewebespezifische virale Vektor den Ursprung der DNA-Replikation und im wesentlichen die späte Genregion des lymphotropen Papova-Virus (LPV) umfaßt und LPV-permissive Zellen, die die frühe Genregion von LPV stabil exprimieren, als Wirt fungieren.

3. Verfahren zur Expression von Fremd-DNA, dadurch gekennzeichnet, daß diese Fremd-DNA virale DNA in einem Wirts-Vektorsystem nach Anspruch 1 ersetzt.

4. Verfahren nach Anspruch 3, dadurch gekennzeichnet, daß die Fremd-DNA für ein Protein kodiert, das für die Wirtszelle toxisch ist.

5. Verfahren zur Herstellung eines Vektors für ein Wirts-Vektorsystem nach Anspruch 1, dadurch gekennzeichnet, daß in das Genom von LPV eine singuläre Schnittstelle zwischen früher und später Genregion eingeführt wird und das etwa 3 kb umfassende Segment von der NcoI-Schnittstelle bis zu der eingeführten Schnittstelle verwendet wird.

6. Verfahren zur Herstellung von Wirtszellen für ein Wirts-Vektorsystem nach Anspruch 1, dadurch gekennzeichnet, daß man eine LPV-permissive Zellinie mit einem LPV-DNA-Fragment transfiziert, das das 4,2 kb NcoI-Segment umfaßt.

## FIG. 1

BamHI NcoI Smal/XmaI

Ap
ori
ori
pBR322
pLPVK38
9,63 kb
T-AG
BamHI
(1)

$\xrightarrow{\text{BamHI}\ /\ \text{Ligase}}$

BamHI NcoI XmaI
ori XmaI
t-AG VP2 VP3
LPVK 38
5,27 kb
T-AG
T-AG
VP1
(2)

(2) $\xrightarrow{\text{XmaI}}$ (XmaI)—(4695 bp)—(XmaI)  (3)

pUC 19 $\xrightarrow{\text{XmaI}}$ (4)

(3) + (4) $\xrightarrow{\text{Ligase}}$

NcoI XmaI SalI
t-AG ori ori pUC19
T-AG pL3 Ap (5)
7,38 kb
T-AG EcoRI
VP1 XmaI

(5) $\xrightarrow[\text{SalI}]{\text{NcoI}}$ 
TCGAC ≠ CCATG
AGCTG   GGTAC
(SalI⁻) (NcoI⁻)
Klenow-P    (6)

HCMV AD169—PstI—m—Fragment $\xrightarrow[\text{HincII}]{\text{AvaII}}$ 
GTCC               GTC
CAGG —(HCMV)— CAG
Klenow-P (AvaII⁻)     (7)  (HincII)

(6) + (7) $\xrightarrow{\text{Ligase}}$

NcoI/AvaII (HincII7SalI⁻)
P/E
t-AG
T-AG pL12
7,48 kb pUC 19
Ap
VP1 EcoRI
SmaI/Xma
(8)

$(8) \xrightarrow[\text{Sma I}]{\text{Eco RI}} \overset{\text{AATTC}}{\underset{\text{G}}{}} - (\text{pUC 19}) (\text{P/E}) (\overrightarrow{\text{T-AG}}) - \overset{\text{CCC}}{\underset{\text{GGG}}{}} \quad (9)$

(EcoRI)  (SmaI)

## FIG.1a

$\text{pSV2 Neo} \xrightarrow[\substack{\text{2. Klenow-P.} \\ \text{3. Eco RI}}]{\text{1. Acc I}} (\text{Acc I}^-) - (\overrightarrow{\text{Neo}}) - \overset{\text{G}}{\underset{\text{CTTAA}}{}} \quad (10)$

(Eco RI)

NcoI/AvaII

(9) + (10) →Ligase→ **pL 30** 10,9 kb (11)

P/E · t-AG · T-AG · VP1 · Neo · Ap · pUC 19 · EcoRI

---

pSP 64 →BamHI→ (12)

(2) →BclI→ (13)

(12) + (13) ──→ NcoI

(14)

## FIG. 2

(BamHI⁻/BclI⁻)

HincII · T-AG · ori · pSP64 · Ap · HincII · T · VP1 · VP2 · VP3 · ori

**pL9** 8,33 kb

(BamHI⁻/BclI⁻)

$(14) \xrightarrow{\text{Hinc II}} (\text{Hinc II}) - (\text{VP, ori}) - (\text{Hinc II}) \quad (15) +$

4912 bp

$(\text{Hinc II}) - (\text{pSP64}) - (\text{Hinc II}) \quad (16)$

3415 bp

pUC 19 $\xrightarrow{\text{Bam HI}}$ (17)

(2) $\xrightarrow{\text{Bam HI}}$ (18)

(17) + (18) $\xrightarrow{\text{Ligase}}$

(19) $\xrightarrow[\text{Ligase}]{\text{Apa I}}$ pL 24 (20)

(20) $\xrightarrow{\text{Bam HI}}$ (21)

M13mp 18 $\xrightarrow{\text{Bam HI}}$ (22)

(21) + (22) $\xrightarrow{\text{Ligase}}$

```
2832                2852
 |                   |
TCTTCAATTAGAATTCCATGC
         └────┘
          EcoRI          (24)
```

(23) ssDNA + (24) $\xrightarrow[\substack{\text{Klenow-P.}\\ \text{Ligase}}]{\text{Hybrid.,}}$

**FIG. 3**

Bam HI

pUC 19

T-AG

pLPVK 38-1
7,96 kb

VP

ori

Bam HI

NcoI

ApaI

(19)

Bam HI

T-AG

mL 58
12,28 kb

M13mp 18

VP

ori

ApaI

NcoI

Bam HI

(23)

Bam HI

EcoRI

T-AG

mL 60
12,28 kb

M13mp 18

VP

ori

ApaI

NcoI

Bam HI

(25)

FIG. 3a

$(25) \xrightarrow[\text{EcoRI}]{\text{BamHI}} (\text{BamHI}) - \text{ori} - \text{VP} - \text{EcoRI}) \ (26)$

$\textbf{pUC 19} \xrightarrow[\text{EcoRI}]{\text{BamHI}} (27)$

$(26) + (27) \xrightarrow{\text{Ligase}}$

pL 61
5,51kb
(28)

$(28) \xrightarrow[\text{NcoI}]{\text{BamHI}} (\text{NcoI}) - \text{ori} - \text{VP} - \text{pUC 19} - (\text{BamHI}) \quad (29)$

$(\text{BamHI}) - X - (\text{NcoI}) \ (30)$

$(29) + (30) \xrightarrow{\text{Ligase}}$

pLX
≦ 7,8kb
(31)

$(31) \xrightarrow[\substack{\text{BamHI} \\ \text{Transfection}}]{\text{EcoRI}}$

LPV X
≦ 5,2kb
(32)

| EINSCHLÄGIGE DOKUMENTE | | | EP 87113428.4 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | WO - A1 - 83/02 393 (ANIMAL VACCINE RESEARCH CORPORATION)<br><br>* Ansprüche 1,4,8 *<br><br>-- | 1,3 | C 12 N 15/00<br>C 12 N 5/00<br>A 61 K 37/02 |
| D,A | VIROLOGY, vol. 143, no. 1, May 1985 (New York)<br><br>M. PAWLITA et al. "Complete DNA Sequence of Lymphotropic Papova-virus: Prototype of a New Species of the Polyomavirus Genus" pages 196-211<br><br>* Gesamt *<br><br>-- | 1,2 | |
| A | WO - A1 - 84/04 540 (THE WISTAR INSTITUTE OF ANATOMY AND BIOLOGY)<br><br>* Ansprüche 2,4,21 *<br><br>-- | 1-3,5 | RECHERCHIERTE SACHGEBIETE (Int. Cl.4) |
| D,A | EP - A1 - 0 173 177 (BEHRINGWERKE AKTIENGESELLSCHAFT)<br><br>* Patentansprüche *<br><br>---- | 3,5 | C 12 N<br>A 61 K |

Der vorliegende Recherchenbericht wurde für alle Patentansprüche erstellt.

| Recherchenort | Abschlußdatum der Recherche | Prüfer |
|---|---|---|
| WIEN | 10-12-1987 | WOLF |